# EUROPÄISCHE PATENTSCHRIFT

(11) **EP 1 296 969 B1**
(45) Veröffentlichungstag und Bekanntmachung des Hinweises auf die Patenterteilung: **15.04.2009**
(21) Anmeldenummer: 01945339.8
(22) Anmeldetag: 05.07.2001
(51) Int. Cl.: C07D 301/32

(54) **VERFAHREN ZUR HERSTELLUNG VON PROPYLENOXID**
METHOD FOR THE PRODUCTION OF PROPYLENE OXIDE
PROCEDE DE PRODUCTION D'OXYDE DE PROPYLENE

(30) Priorität: 06.07.2000 DE 10032885
(43) Veröffentlichungstag der Anmeldung: 02.04.2003
(73) Patentinhaber: BASF SE, 67056 Ludwigshafen (DE)
(72) Erfinder: TELES, Joaquim, Henrique, 67166 Otterstadt (DE); REHFINGER, Alwin, 67112 Mutterstadt (DE); BASSLER, Peter, 68519 Viernheim (DE); BERG,Anne, B-2170 Merksem (DE); REIBER, Norbert, 68259 Mannheim (DE); RUDOLF, Peter, 68526 Ladenburg (DE)
(74) Vertreter: Isenbruck, Günter
(86) Internationale Anmeldenummer: PCT/EP2001/007717
(87) Internationale Veröffentlichungsnummer: WO 2002/002545

(56) Entgegenhaltungen:
- EP-A- 0 524 816
- DD-B- 215 084
- US-A- 3 350 417
- US-A- 3 715 284
- US-A- 3 838 020
- US-A- 3 881 996
- US-A- 5 106 458
- US-A- 5 107 002
- US-A- 5 489 366

## Beschreibung

Die vorliegende Erfindung betrifft ein Verfahren zur Herstellung von Propylenoxid, bei dem Methanol eingesetzt wird. Im Rahmen des erfindungsgemäßen Verfahrens wird Methanol aufgearbeitet und dabei aus einem Gemisch, das Methanol und Methylformiat enthält, abgetrennt.

In den gängigen Verfahren des Standes der Technik, in denen Propylenoxid in Gegenwart von Methanol hergestellt wird, entsteht im Regelfall in mindestens einer Verfahrensstufe aus Methanol und/oder aus Propylenoxid Methylformiat. Insbesondere tritt dann das Problem auf, daß das Wertprodukt Propylenoxid von dem unerwünschten Nebenprodukt Methylformiat abgetrennt werden muß und aufgrund der dicht beieinanderliegenden Siedepunkte dieser beiden Verbindungen ein hoher apparativer Aufwand zur Trennung erforderlich ist. Bei den diesbezüglich im Normalfall eingesetzten Destillationsverfahren sind demgemäß beispielsweise Extraktivdestillationsverfahren notwendig, um die geforderten Wertproduktreinheiten zu gewährleisten.

Solche aufwendigen Verfahren zur Abtrennung von Methylformiat aus Propylenoxid sind beispielsweise in der US-A 5,107,002 oder in der US-A 5,106,458 beschrieben.

Eine der vorliegenden Erfindung zugrundeliegende Aufgabe war es daher, ein Verfahren bereitzustellen, das einen geringeren apparativen Aufwand ermöglicht und eine effizientere und kostengünstigere Herstellung von Propylenoxid gewährleistet.

Daher betrifft die vorliegende Erfindung ein Verfahren zur Herstellung von Propylenoxid in Gegenwart von Methanol, in dem aus einem Gemisch, umfassend Propylenoxid und Methanol, Propylenoxid abgetrennt wird und das resultierende Gemisch, umfassend Methanol, aufgearbeitet wird, dadurch gekennzeichnet, daß bei der Aufarbeitung aus einem Gemisch, umfassend Methanol und Methylformiat, Methanol abgetrennt wird.

Die Abtrennung des Methanols aus dem Gemisch, umfassend Methylformiat und Methanol, kann hierbei prinzipiell gemäß allen denkbaren Verfahren bewerkstelligt werden, solange gewährleistet ist, daß die Reinheit des abgetrennten Methanols den gestellten Anforderungen genügt.

Unter anderem sind hierbei chemische Methoden zu nennen. Beispielsweise ist es etwa möglich, das Gemisch, umfassend Methanol und Methylformiat, mit einem geeigneten basischen Ionentauscher in Kontakt zu bringen, wodurch Methanol entsteht und das Formiat am Ionentauscher verbleibt. Dieses Verfahren ist unter anderem in der US-A 5,107,002 beschrieben.

Weiter kann das Gemisch, umfassend Methanol und Methylformiat, mit einer Base behandelt werden, wobei das Methylformiat hydrolisiert wird. Dabei sind sämtliche Basen verwendbar, durch die die Hydrolyse des Methylformiats erreicht werden kann. Bevorzugt werden starke Basen eingesetzt. Als besonders bevorzugte Basen sind im Rahmen der vorliegenden Erfindung Salze von Säuren zu nennen, die schwächere Säuren als Ameisensäure sind. Unter anderem bevorzugt sind hierbei etwa Alkali- und Erdalkalihydroxide oder Alkalisalze von Alkoholen oder Phenolen zu nennen. Selbstverständlich können auch Mischungen aus zwei oder mehr dieser Basen eingesetzt werden.

Weiter bevorzugt sind zur Abtrennung von Methanol aus dem Gemisch, umfassend Methanol und Methylformiat, physikalische Methoden wie beispielsweise Destillationsverfahren.

Unter diesen sind beispielsweise Extraktivdestillationsverfahren möglich, wie sie aus dem Stand der Technik bekannt und beispielsweise in der oben genannten US-A 5,107,002 aufgeführt sind.

Bevorzugt werden jedoch Destillationsverfahren eingesetzt, die apparativ weniger aufwendig zu realisieren sind als die genannten Extraktionsdestillationsverfahren.

Bevorzugt wird ein Destillationsverfahren, in dem eine oder mehrere Kolonnen, weiter bevorzugt eine Kolonne, eingesetzt wird. Wird eine Kolonne eingesetzt, so weist diese mindestens 5, bevorzugt mindestens 10 und insbesondere mindestens 20 theoretische Böden auf.

Die Drücke, bei denen bevorzugt gearbeitet wird, liegen im allgemeinen im Bereich von 0,2 bis 50 bar, bevorzugt im Bereich von 1,5 bis 30 bar und insbesondere im Bereich von 2,0 bis 20 bar.

Die Kopf- und Sumpftemperaturen werden vom gewählten Druck eindeutig bestimmt. In einer besonders bevorzugten Ausführungsform wird diese Kolonne, die ungefähr 20 theoretische Trennstufen aufweist, im allgemeinen im Bereich von 0,2 bis 50 bar, bevorzugt im Bereich von 1,5 bis 30 bar und ganz besonders bevorzugt im Bereich von 2,0 bis 20 bar betrieben. Als Kopfprodukt erhält man ein Gemisch, umfassend Methylformiat und einen geringen Anteil des im Feed enthaltenen Methanols. Im allgemeinen weist das erhaltene Gemisch einen Methanolanteil von weniger als 80 Gew.-%, bevorzugt von weniger als 50 Gew.-% und besonderes bevorzugt von weniger als 20 Gew.-% auf.

Daher betrifft die vorliegende Erfindung auch ein Verfahren, wie oben beschrieben, das dadurch gekennzeichnet ist, daß die Abtrennung des Methanols aus dem Gemisch, umfassend Methanol und Methylformiat, in einer Destillationskolonne mit mindestens 5 theoretischen Böden bei Drücken im Bereich von 0,2 bis 50 bar durchgeführt wird.

Weiter ist es denkbar, daß das Gemisch, umfassend Methanol und Methylformiat, neben Methylformiat zusätzlich weitere Komponenten aufweist. Der Begriff "Komponenten" bezeichnet hierbei sowohl reine Verbindungen als auch Azeotrope, die einen Siedepunkt aufweisen, der niedriger ist als der Siedepunkt von Methanol. Als solche Komponenten sind unter anderem beispielhaft Acetaldehyd, 1,1-Dimethoxyethan, Propionaldehyd, 1,1-Dimethoxypropan, Aceton oder 2,4-Dimethyl-1,3-dioxolan zu nennen. Diese können ebenfalls im Rahmen der Aufarbeitung aus dem Gemisch abgetrennt werden.

So ist es möglich, diese Nebenprodukte vor der Trennung des Methanols von Methylformiat durch eine oder mehrere geeignete physikalische oder chemische Methoden aus dem Gemisch abzutrennen. Ebenso ist es möglich, zuerst Methanol aus dem Gemisch abzutrennen, wobei ein Gemisch resultieren kann, das Methanol und mindestens eine Verunreinigung enthält. In diesem Fall können sich an die Abtrennung von Methanol aus dem Gemisch eine oder mehrere Abtrennstufen anschließen, in denen Methanol von der mindestens einen Verunreinigung abgetrennt wird. Ebenso kann nach der Abtrennung von Methanol aus dem Gemisch ein Gemisch resultieren, das Methylformiat und eine oder mehrere Verunreinigungen enthält. Auch dieses kann, falls erforderlich, durch eine oder mehrere geeignete physikalische oder chemische Methoden in seine Bestandteile getrennt werden. Die Bestandteile können dann getrennt oder zusammen einem oder mehreren weiteren Verfahren als Edukte zugeführt oder thermisch verwertet werden.

Je nach chemischer Natur der Verunreinigungen ist es auch möglich, Methanol derart aus dem Gemisch abzutrennen, daß sowohl Methylformiat als auch die mindestes eine Verunreinigung in einem einzigen Verfahrensschritt von Methanol abgetrennt werden.

Durch die wie oben beschriebene, erfindungsgemäß bevorzugt eingesetzte Destillation wird dabei eine Methanolfraktion erhalten, die einen Gehalt an Methylformiat von im allgemeinen weniger als 500 ppm, bevorzugt weniger als 100 ppm und insbesondere bevorzugt weniger als 20 ppm aufweist.

Demgemäß betrifft die vorliegende Erfindung auch ein Verfahren, wie oben beschrieben, das dadurch gekennzeichnet ist, daß das im Rahmen der Aufarbeitung abgetrennte Methanol einen Gehalt an Methylformiat von weniger als 500 ppm aufweist.

In Abhängigkeit von den Anforderungen, die an die Reinheit der Methanolfraktion gestellt werden, können Reste von anderen Komponenten wie beispielsweise Acetaldehyd, 1,1-Dimethoxyethan, Propionaldehyd, 1,1-Dimethoxypropan, Aceton oder 2,4-Dimethyl-1,3-dioxolan, die im Anschluß an die destillative Aufarbeitung in der Methanolfraktion verbleiben, durch eine oder mehrere geeignete Maßnahmen wie beispielsweise eine oder mehrere weitere Destillationen von Methanol abgetrennt werden.

Im allgemeinen ist es völlig ausreichend, wenn die Konzentration jeder einzelnen Nebenkomponente im Methanol unter 1 Gew.-% liegt und die Summe aller Nebenkomponenten 5 Gew.-% nicht überschreitet.

Das derart von Methylformiat abgetrennte Methanol kann wiederverwendet werden, wobei es prinzipiell denkbar ist, das Methanol in das Verfahren zur Herstellung des Propylenoxides rückzuführen oder bei Bedarf einem davon unterschiedlichen Verfahren, in dem Methanol als Lösungsmittel oder als Edukt oder in sonstiger Funktion benötigt wird, zuzuführen. Selbstverständlich ist es denkbar, den Methanolstrom, der aus der erfindungsgemäßen Abtrennung resultiert, in zwei oder mehr Ströme aufzuteilen und jeden Strom einem anderen Verfahren zuzuführen.

In einer besonders bevorzugten Ausführungsform des erfindungsgemäßen Verfahrens wird das Methanol, das von Methylformiat und gegebenenfalls von einem oder mehreren Nebenproduk-ten oder Verunreinigungen getrennt wurde, wie oben beschrieben in das Verfahren zur Herstellung des Propylenoxides rückgeführt. Unter anderem bevorzugt wird das Methanol in einen Puffertank gepumpt und daraus in das Verfahren eingeschleust.

Demgemäß betrifft die vorliegende Erfindung auch ein Verfahren, wie oben beschrieben, das dadurch gekennzeichnet ist, daß das im Rahmen der Aufarbeitung von Methylformiat abgetrennte Methanol in das Verfahren rückgeführt wird.

Die Herstellung des Propylenoxides kann prinzipiell nach sämtlichen Verfahren durchgeführt werden, die in Gegenwart von Methanol durchgeführt werden.

In einer bevorzugten Ausführungsform des erfindungsgemäßen Verfahrens wird das Propylenoxid in Gegenwart von Methanol in mindestens einer Reaktionsstufe aus Propen und Wasserstoffperoxid hergestellt, wobei ein Gemisch resultiert, das Methanol, Propylenoxid und Wasser enthält.

Die Umsetzung von Propen mit Wasserstoffperoxid kann dabei nach sämtlichen geeigneten Methoden erfolgen. Beispielsweise kann die Propylenoxidherstellung in einem Batch-Prozeß oder kontinuierlich durchgeführt werden.

Hinsichtlich der kontinuierlichen Verfahren sind wiederum sämtliche geeigneten Reaktoranordnungen denkbar. So kann beispielsweise das Propylenoxid in einer Kaskade aus zwei oder mehr Reaktores, die seriell miteinander verbunden sind, hergestellt werden. Ebenso sind Verfahren denkbar, in denen parallel angeordnete Reaktoren eingesetzt werden. Auch Kombinationen dieser Verfahren sind möglich. Im Falle, daß zwei oder mehr Reaktoren in Serie geschaltet sind, können zwischen den Reaktoren auch geeignete Zwischenbehandlungen wie vorgesehen werden. Unter anderem sei in diesem Zusammenhang auf die PCT/EP99/05740 und die DE-A 100 15 246.5 verwiesen, die bezüglich Reaktoranordnung und Zwischenbehandlung vollumfänglich durch Bezugnahme in den Kontext der vorliegenden Anmeldung einbezogen werden.

Weiter können im Laufe der Herstellung des Propylenoxides aus Propen und Wasserstoffperoxid während des Verfahrens Temperatur und Druck des Reaktionsmediums geändert werden. Ebenso können der pH-Wert und die Temperatur des Reaktionsmediums geändert werden. Weiter ist es möglich, zusätzlich zu pH-Wert und Temperatur des Reaktionsmediums zusätzlich den Druck zu ändern, unter dem die Reaktion stattfindet. Diesbezüglich sei auf die DE-A 199 36 547.4 verwiesen, die diesbezüglich vollumfänglich durch Bezugnahme in den Kontext der vorliegenden Anmeldung einbezogen wird.

Das Gemisch, das aus der Herstellung des Propylenoxides aus Propen und Wasserstoffperoxid resultiert und Methanol, Propylenoxid und Wasser umfaßt, wird im Rahmen des erfindungsgemäßen Verfahrens bevorzugt derart aufgearbeitet, daß zunächst Propylenoxid abgetrennt wird.

In einer besonders bevorzugten Ausführungsform betrifft die vorliegende Erfindung daher ein Verfahren, wie oben beschrieben, in dem
(i) in mindestens einer Reaktionsstufe Propylenoxid aus Propen und Wasserstoffperoxid in Gegenwart von Methanol hergestellt wird, wobei ein Gemisch (Gi) erhalten wird, das Methanol, Propylenoxid und Wasser umfaßt,
(ii) aus dem Gemisch (Gi) das Propylenoxid abgetrennt wird, wobei ein Gemisch (Gii) erhalten wird, das Methanol und Wasser umfaßt,
(iii) aus dem Gemisch (Gii) Wasser abgetrennt wird, wobei ein Gemisch (Giii) erhalten wird, das Methanol und Methylformiat umfaßt,
(iv) aus dem Gemisch (Giii) Methanol abgetrennt und
(v) das gemäß (iv) abgetrennte Methanol in (i) rückgeführt wird.

Die Abtrennung von Wasser gemäß (iii) aus dem Gemisch (Gii) wird im erfindungsgemäßen Verfahren bevorzugt destillativ durchgeführt, wobei eine oder auch mehrere Destillationskolonnen verwendet werden können. Bevorzugt werden eine oder zwei Destillationskolonnen eingesetzt. Im Falle, daß keine Wärmerückgewinnung nötig ist, wird bevorzugt eine Destillationskolonne eingesetzt. Zwei oder auch mehr Destillationskolonnen werden bevorzugt dann eingesetzt, wenn eine besonders gute Wärmeintegration im Verfahren gewährleistet werden soll.

Hinsichtlich der physikalischen Parameter wie Temperatur oder Druck bestehen bei der destillativen Abtrennung von Wasser aus dem Gemisch (Gii) keine besonderen Einschränkungen. Wird im erfindungsgemäßen Verfahren zur Abtrennung des Wassers aus dem Gemisch (Gii) nur eine Kolonne eingesetzt, so weist diese bevorzugt mindestens 5, bevorzugt mindestens 20 und weiter bevorzugt mindestens 30 theoretische Böden auf. Bevorzugt wird die Destillation bei Drücken im Bereich von 0,5 bis 40 bar, bevorzugt von 1,0 bis 20 bar und besonders bevorzugt von 2,0 bis 15 bar durchgeführt.

Werden im erfindungsgemäßen Verfahren zur Abtrennung des Wassers aus dem Gemisch (Gii) zwei Kolonnen eingesetzt, dann werden die Drücke so gewählt, daß mit der Kondensationswärme am Kopf der Kolonnen andere Prozeßströme aufgeheizt werden können. Dies wird beispielsweise dadurch erreicht, daß der Kondensator mindestens einer Kolonne mit beispielsweise Wasser gekühlt wird und das aus der Kühlung resultierende Warmwasser oder der aus der Kühlung resultierende Dampf zur Beheizung eines oder mehrerer Schritte des erfindungsgemäßen Verfahrens oder auch eines oder mehrerer anderer Verfahren eingesetzt wird.

Bevorzugt wird die erste Destillationskolonne bei Drücken im Bereich von 0,5 bis 40 bar und bevorzugt von 1 bis 20 bar durchgeführt. In einer möglichen Ausführungsform wird die erste Kolonne bei einem höheren Druckniveau betrieben als die zweite Kolonne. In diesem Fall wird der Sumpf der zweiten Kolonne mit dem Kondensat der ersten Kolonnen beheizt. In einer bevorzugten Ausführungsform wird die erste Kolonne bei einem niedrigeren Druckniveau betrieben als die zweite Kolonne. In diesem Fall wird der Sumpf der ersten Kolonne mit dem Kondensat der zweiten Kolonne beheizt.

In einer besonders bevorzugten Ausführungsform werden die erste Kolonne bei Drücken im Bereich von 4 bis 9 bar und weiter bevorzugt im Bereich von 6 bis 8 bar und die zweite im Bereich von 11 bis 16 bar und weiter bevorzugt im Bereich von 12 bis 14 bar betrieben. Im Kopf der ersten Kolonne werden im allgemeinen 20 bis 80 %, bevorzugt 30 bis 70 % und besonders bevorzugt 40 bis 60 % des im Gemisch (Gii) enthaltenen Methanols zusammen mit Methylformiat über Kopf abgetrennt. Das Gemisch, das über Sumpf aus der ersten Kolonne erhalten wird, wird komprimiert und als Feed für die zweite Kolonne verwendet. Das Kopfprodukt der zweiten Kolonne umfaßt das restliche Methanol und Methylformiat und das Sumpfprodukt umfaßt Wasser. Das Kopfprodukt der ersten Kolonne und das Kopfprodukt der zweiten Kolonne werden zum Gemisch (Giii) vereinigt.

Sowohl im Fall der Wasserabtrennung in zwei Kolonnen als auch im Fall der Wasserabtrennung in einer Kolonne werden die Trennbedingungen besonders bevorzugt so gewählt, dass der Wassergehalt im Gemisch (Giii) im allgemeinen weniger als 3 Gew.-%, bevorzugt weniger als 1 Gew.-% und besonders bevorzugt weniger als 0,3 Gew.-% beträgt. Weiter bevorzugt werden die Trennbedingungen derart gewählt, daß der Methanolgehalt im Sumpfabzug weniger als 5 Gew.-%, bevorzugt weniger als 1 Gew.-% und besonders bevorzugt weniger als 0,2 Gew.-% beträgt.

Der Sumpfabzug kann daneben als weitere Komponenten unter anderem beispielsweise Methoxypropanole, Propylenglykol, Ameisensäure, Dipropylenglykolemonomethylether, Formaldehyd enthalten.

Daher betrifft die vorliegende Erfindung auch ein Verfahren, wie oben beschrieben, das dadurch gekennzeichnet ist, daß das Wassers gemäß (iii) destillativ abgetrennt wird, wobei
(w) aus dem Gemisch (Gii) in einer ersten Destillationskolonne über Kopf ein Gemisch (Gw) abgetrennt wird, das hauptsächlich Methanol und Methylformiat umfaßt,
(x) das aus der ersten Destillationskolonne über Sumpf erhaltene Gemisch als Feed einer zweiten Destillationskolonne zugeführt wird,
(y) aus der zweiten Destillationskolonne über Kopf ein Gemisch (Gy) erhalten wird, das hauptsächlich Methanol und Methylformiat umfaßt, und
(z) die Gemische (Gw) und (Gy) unter Erhalt des Gemisches (Giii) vereinigt werden.

Wie oben beschrieben, wird erfindungsgemäß aus dem Gemisch (Gi) zunächst das in (i) hergestellte Propylenoxid abgetrennt, bevor aus dem resultierenden Gemisch (Gii) Wasser abgetrennt wird. Auch diese Abtrennung kann generell durch sämtliche geeigneten Verfahren erfolgen, wobei wiederum eine destillative Abtrennung bevorzugt ist.

Enthält hierbei das Gemisch (Gi) kein oder vernachlässigbar wenig Propen, das in (i) nicht umgesetzt wurde, so ist es prinzipiell möglich, aus dem Gemisch (Gi) in (ii) direkt Propylenoxid abzutrennen.

Im allgemeinen wird jedoch im erfindungsgemäßen Verfahren das Gemisch (Gi) soviel nicht-umgesetztes Propen enthalten, daß dessen Abtrennung notwendig ist. In diesem Fall wird das Verfahren bevorzugt derart durchgeführt, daß in einem ersten Schritt nicht-umgesetztes Propen aus dem Gemisch (Gi) abgetrennt wird und aus dem resultierenden Gemisch, das Propylenoxid enthält, das Propylenoxid abgetrennt wird.

Demgemäß betrifft die vorliegende Erfindung auch ein Verfahren, wie oben beschrieben, das dadurch gekennzeichnet ist, daß die Abtrennung des Propylenoxids gemäß (ii) in mindestens zwei Stufen (a) und (b) erfolgt, wobei
(a) aus dem Gemisch (Gi), das zusätzlich zu Methanol, Propylenoxid und Wasser in (i) nicht umgesetztes Propen umfaßt, Propen unter Erhalt eines Gemisches (Ga), umfassend Methanol, Propylenoxid und Wasser, abgetrennt wird und
(b) aus dem Gemisch (Ga) Propylenoxid unter Erhalt des Gemisches (Gii) abgetrennt wird.

Während für diese Abtrennungen gemäß (a) und (b) wiederum sämtliche geeigneten Methoden möglich sind, sind destillative Verfahrensführungen bevorzugt.

Prinzipiell können, wie oben beschrieben, beispielsweise Propen und Propylenoxid in einer einzigen Destillationskolonne aus dem Gemisch (Gi) abgetrennt werden, wobei beispielsweise das Propylenoxid über den Seitenabzug und das Propen über Kopf abgetrennt werden, wobei dann über Sumpf ein Gemisch, umfassend Methanol und Wasser, erhalten wird.

Bevorzugt wird jedoch der Einsatz von mindestens zwei voneinander getrennten Kolonnen, wobei in mindestens einer Kolonne das Propen bevorzugt über Kopf abgezogen wird und über Sumpf ein Gemisch (Ga) erhalten wird, das Propylenoxid, Methanol und Wasser enthält. In mindestens einer weiteren Kolonne wird aus dem Gemisch (Ga) Propylenoxid bevorzugt über Kopf abgetrennt, wobei über Sumpf das Gemisch (Gii) erhalten wird.

Bevorzugt ist ein Verfahren, in dem die destillative Abtrennung gemäß (a) in einer Kolonne mit im allgemeinen mindestens 5, bevorzugt mindestens 10 und besonders bevorzugt mindestens 15 theoretischen Böden bei Drücken im Bereich von im allgemeinen 0,2 bis 25 bar, bevorzugt von 0,5 bis 5 bar und besonders bevorzugt bei ungefähr 1 bar erfolgt. Die Temperatur, bei der Propen über Kopf abgetrennt wird, liegt hierbei besonders bevorzugt bei ungefähr 25 °C bei dem besonders bevorzugten Druck von ungefähr 1 bar. Die Temperatur, bei der das Gemisch, umfassend Methanol, Wasser und Propylenoxid über Sumpf abgetrennt wird, liegt hierbei besonders bevorzugt bei ungefähr 63 °C bei dem besonders bevorzugten Druck von ungefähr 1 bar.

Was die destillative Abtrennung gemäß (b) anbelangt, so finden im erfindungsgemäßen Verfahren bevorzugt Verfahrensführungen Anwendung, bei denen in einer Kolonne mit im allgemeinen mindestens 20, bevorzugt mindestens 40 und besonders bevorzugt mindestens 60 theoretischen Böden bei Drücken von im allgemeinen im Bereich von 0,3 bis 10 bar, bevorzugt von 0,5 bis 5 bar und besonders bevorzugt von 0,6 bis 1,2 bar gearbeitet wird. Die Temperatur, bei der Propylenoxid über Kopf abgetrennt wird, liegt hierbei bei einem Druck von ungefähr 0,75 bar bei ungefähr 26 °C. Die Temperatur, bei der das Gemisch, umfassend Methanol und Wasser über Sumpf abgetrennt wird, liegt hierbei bei einem Druck von ungefähr 0,75 bar bei ungefähr 67 °C.

Daher betrifft die vorliegende Erfmdung auch ein Verfahren, wie oben beschrieben, das dadurch gekennzeichnet ist, daß die Abtrennungen gemäß (a) und (b) destillativ bei
(a) einem Druck im Bereich von 0,2 bis 25 bar in einer Kolonne mit mindestens 5 theoretischen Böden und
(b) einem Druck im Bereich von 0,3 bis 10 bar in einer Kolonne mit mindestens 20 theoretischen Böden
erfolgen.

Das gemäß (a) abgetrennte Propen wird in einer besonders bevorzugten Ausführungsform des erfindungsgemäßen Verfahrens wieder als Edukt in (i) rückgeführt. Hierbei tritt unter Umständen das Problem auf, daß beim Abtrennen des Propens als Leichtsiederfraktion, wie oben beschrieben, sich in der Leichtsiederfraktion Sauerstoff in einer Konzentration ansammeln kann, die die Leichtsiederfraktion zu einem zündfähigen Gemisch macht. Dadurch kann ein ernstes Sicherheitsrisiko entstehen, wenn Propen destillativ von der Leichtsiederfraktion abgetrennt wird und in (i) rückgeführt wird. Dieses Problem kann beispielsweise dadurch gelöst werden, daß Propen destillativ aus dem Leichtsiedergemisch entfernt wird und im oberen Teil der dafür verwendeten Trenneinrichtung ein inerter Stoff mit einem Siedepunkt, der niedriger als der des Propens ist, bevorzugt Methan, in einer solchen Menge zuzugeben wird, daß der Sauerstoff bis zu einer Konzentration verdünnt ist, bei der das Gemisch nicht mehr zündfähig ist. Dieses Verfahren ist beispielsweise in der EP-B 0 719 768 beschrieben. Bevorzugt wird das Problem jedoch dadurch gelöst, daß ein Verfahren zur Aufarbeitung eines Gemisches, umfassend Propen und Sauerstoff, angewendet wird, in dem Sauerstoff nicht-destillativ aus dem Gemisch unter Erhalt eines weiteren Gemisches entfernt wird und aus dem weiteren Gemisch das Propen destillativ abgetrennt wird. Dieses Verfahren ist in der DE-A 100 01 401.1 beschrieben, die diesbezüglich vollumfänglich durch Bezugnahme in den Kontext der vorliegenden Anmeldung einbezogen wird.

Prinzipiell kann gemäß des oben beschriebenen Verfahrens Methanol als Lösungsmittel eingesetzt und neben Methanol ein oder mehrere weitere geeignete Lösungsmittel verwendet werden. Dieses mindestens eine weitere Lösungsmittel kann, wie Methanol, ebenfalls aufgearbeitet und in das Verfahren rückgeführt werden. Solche weiteren Lösungsmittel sind unter anderem
- Wasser,
- Alkohole, bevorzugt niedere Alkohole, weiter bevorzugt Alkohole mit weniger als 6 Kohlenstoffatomen wie beispielsweise Ethanol, Propanole, Butanole, Pentanole,
- Diole oder Polyole, bevorzugt solche mit weniger als 6 Kohlenstoffatomen,
- Ether wie beispielsweise Diethylether, Tetrahydrofuran, Dioxan, 1,2-Diethoxyethan, 2-Methoxyethanol,
- Ester wie beispielsweise Methylacetat oder Butyrolacton,
- Amide wie beispielsweise Dimethylformamid, Dimethylacetamid, N-Methylpyrrolidon,
- Ketone wie beispielsweise Aceton,
- Nitrile wie beispielsweise Acetonitril
- oder Gemische aus zwei oder mehr der vorgenannten Verbindungen.

Als Katalysatoren zur Umsetzung des Propens zu Propylenoxid sind prinzipiell alle, bevorzugt alle heterogenen Katalysatoren denkbar, die für die jeweilige Umsetzung geeignet sind. Bevorzugt werden dabei Katalysatoren verwendet, die ein poröses oxidisches Material wie z.B. einen Zeolith umfassen. Vorzugsweise werden Katalysatoren eingesetzt, die als poröses oxidisches Material ein Titan-, Vanadium-, Chrom-, Niob- oder Zirkonium-haltigen Zeolith umfassen.

Im besonderen existieren Zeolithe, die kein Aluminium enthalten und bei denen im Silikatgitter anstelle des Si(IV) teilweise Titan als Ti(IV) vorhanden ist. Die Titanzeolithe, insbesondere solche mit einer Kristallstruktur vom MFI-Typ, sowie Möglichkeiten zu ihrer Herstellung sind beschrieben beispielsweise in der EP-A 0 311 983 oder der EP-A 0 405 978.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Dabei sind im einzelnen Titan-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WEI-, WEN-, YUG-, ZON-Sttuktur sowie zu Mischstrukturen aus zwei oder mehr der vorgenannten Strukturen. Denkbar sind für den Einsatz im erfindungsgemäßen Verfahren weiterhin titanhaltige Zeolithe mit der Struktur des UTD-1, CIT-1 oder CIT-5. Als weitere titanhaltige Zeolithe sind solche mit der Struktur des ZSM-48 oder ZSM-12 zu nennen.

Als besonders bevorzugt sind für das erfindungsgemäße Verfahren Ti-Zeolithe mit MFI-, MEL- oder MFI/MEL-Mischstruktur anzusehen. Als weiter bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

Insbesondere bevorzugt wird im erfindungsgemäßen Verfahren ein heterogener Katalysator, der das titanhaltige Silikalit TS-1 umfaßt, verwendet.

Demgemäß betrifft die vorliegende Erfindung auch ein Verfahren,wie oben beschrieben, das dadurch gekennzeichnet ist, daß zur Herstellung des Propylenoxides ein Zeolithkatalysator, bevorzugt ein Titansilikalit-Katalysator und insbesondere ein Titansilikalit-Katalysator der Struktur TS-1 eingesetzt wird.

Dabei ist es im erfindungsgemäßen Verfahren möglich, als Katalysator das poröse oxidische Material an sich zu verwenden. Selbstverständlich ist es jedoch auch möglich, als Katalysator einen Formkörper einzusetzen, der das poröse oxidische Material umfaßt. Dabei können zur Herstellung des Formkörpers, ausgehend von dem porösen oxidischen Material, alle Verfahren gemäß dem Stand der Technik eingesetzt werden.

Sollte es Rahmen des erfindungsgemäßen Verfahrens erforderlich sein, so kann der eingesetzte Katalysator regeneriert werden. Solche Verfahren sind beispielsweise in der DE-A 100 15 246.5 beschrieben, die diesbezüglich durch Bezugnahme in den Kontext der vorliegenden Anmeldung einbezogen wird.

In den folgenden Beispielen wird die vorliegende Erfindung näher erläutert.

### Beispiele

### Vergleichsbeispiel

Ein Gemisch aus Methanol, 30 %iger wäßriger Wasserstoffperoxidlösung und Propen im Massenverhältnis 64,5 : 15,5 : 20 wurde in einem Autoklaven mit TS-1-Pulver bei 0 °C unter Eigendruck solange umgesetzt, bis der Wasserstoffperoxid-Umsatz über 99 % lag.

Anschließend wurde der Reaktorinhalt über eine Steigleitung mit eingebautem Filter, der zur Rückhaltung des Katalysators diente, in eine auf 0 °C gekühlte Blase einer Destillationsapparatur geleitet.

Das Rohprodukt wurde dann destilliert und diejenige Fraktion, die im Bereich von 33 bis 36 °C siedet und das Propylenoxid enthält, gesammelt.

Die Destillation wurde dann fortgeführt und der Schnitt (S) mit einem Siedepunkt im Bereich von 56 bis 66 °C gesammelt. Der Schnitt enthielt im wesentlichen die gesamte Menge an Methanol mit geringen Mengen an Acetaldehyd (ca. 0,3 bis 0,6 Gew.-%), Aceton (ca. 0,1 bis 0,3 Gew.-%), 1,1-Dimethoxyethan (ca. 0,4 bis 0,8 Gew.-%) und Methylformiat (ca. 50 bis 120 ppm). Die Gew.-%-Angaben sind jeweils bezogen auf das Gewicht des Schnittes.

Die oben beschrieben Umsetzung des Propens wurde im Anschluß daran mit dem Methanolschnitt (S) als Lösungsmittel wiederholt. Die Destillation des erhaltenen Rohproduktes ergab einen Propylenoxid-Schnitt (Destillationsfraktion im Bereich von 33 bis 36 °C), der einen Methylformiatgehalt im Bereich zwischen 1000 und 2500 ppm aufwies.

### Erfindungsgemäßes Beispiel

Die erste Umsetzung und die erste Destillation wurden gemäß Vergleichsbeispiel wiederholt.

Der erhaltene Methanolschnitt (S) wurde einer zweiten Destillation unterzogen, wobei eine Kolonne mit mindestens zehn theoretischen Böden eingesetzt wurde. Es wurde solange Kopfprodukt abgenommen, bis die Kopftemperatur 58 °C überstieg. Insgesamt wurden ca. 1 % der eingesetzten Methanol-Fraktion als Kopfprodukt abdestilliert. Das verbleibende Sumpfprodukt enthielt weniger als 10 ppm Methylformiat.

Mit diesem gewonnenen Methanol als Sumpfprodukt wurde die Umsetzung des Propens mit Wasserstoffperoxid wiederholt.

Die Destillation des erhaltenen Rohproduktes ergab einen Propylenoxid-Schnitt (Destillationsfraktion im Bereich von 33 bis 36 °C), der einen Methylformiatgehalt im Bereich von weniger als 10 ppm aufwies.

Der Gehalt an Methylformiat wurde jeweils über Gaschromatographie bestimmt.

## Patentansprüche

1. Verfahren zur Herstellung von Propylenoxid in Gegenwart von Methanol, in dem
(i) in mindestens einer Reaktionsstufe Propylenoxid aus Propen und Wasserstoffperoxid in Gegenwart von Methanol hergestellt wird, wobei ein Gemisch (Gi) erhalten wird, das Methanol, Propylenoxid und Wasser umfaßt,
(ii) aus dem Gemisch (Gi) das Propylenoxid abgetrennt wird, wobei ein Gemisch (Gii) erhalten wird, das Methanol und Wasser umfaßt,
(iii) aus dem Gemisch (Gii) Wasser abgetrennt wird, wobei ein Gemisch (Giii) erhalten wird, das Methanol und Methylformiat umfaßt,
(iv) aus dem Gemisch (Giii) Methanol abgetrennt und
(v) das gemäß (iv) abgetrennte Methanol in (i) rückgeführt wird.

2. Verfahren nach Anspruch 1, **dadurch gekennzeichnet, daß** die Abtrennung des Methanols aus dem Gemisch (Giii), umfassend Methanol und Methylformiat, in einer Destillationskolonne mit mindestens 5 theoretischen Böden bei Drücken im Bereich von 0,2 bis 50 bar durchgeführt wird.

3. Verfahren nach Anspruch 1 oder 2, **dadurch gekennzeichnet, daß** das gemäß (iv) im Rahmen der Aufarbeitung abgetrennte Methanol einen Gehalt an Methylformiat von weniger als 500 ppm aufweist.

4. Verfahren nach einem der Ansprüche 1 bis 3, **dadurch gekennzeichnet, daß** die Abtrennung von Wasser gemäß (iii) aus dem Gemisch (Gii) destillativ erfolgt, wobei eine Destillationskolonne verwendet wird, die mindestens 5 theoretische Böden aufweist, und wobei die Destillation bei Drücken im Bereich von 0,5 bis 40 bar durchgeführt wird.

5. Verfahren nach einem der Ansprüche 1 bis 3, **dadurch gekennzeichnet, daß** das Wassers gemäß (iii) destillativ abgetrennt wird, wobei
(w) aus dem Gemisch (Gii) in einer ersten Destillationskolonne über Kopf ein Gemisch (Gw) abgetrennt wird, das hauptsächlich Methanol und Methylformiat umfaßt,
(x) das aus der ersten Destillationskolonne über Sumpf erhaltene Gemisch als Feed einer zweiten Destillationskolonne zugeführt wird,
(y) aus der zweiten Destillationskolonne über Kopf ein Gemisch (Gy) erhalten wird, das hauptsächlich Methanol und Methylformiat umfaßt, und
(z) die Gemische (Gw) und (Gy) unter Erhalt des Gemisches (Giii) vereinigt werden.

6. Verfahren nach einem der Ansprüche 1 bis 5, **dadurch gekennzeichnet, dass** aus dem Gemisch (Gi) das in (i) hergestellte Propylenoxid destillativ abgetrennt wird.

7. Verfahren nach einem der Ansprüche 1 bis 5, **dadurch gekennzeichnet, daß** die Abtrennung des Propylenoxids gemäß (ii) in mindestens zwei Stufen (a) und (b) erfolgt, wobei
(a) aus dem Gemisch (Gi), das zusätzlich zu Methanol, Propylenoxid und Wasser in (i) nicht umgesetztes Propen umfaßt, Propen unter Erhalt eines Gemisches (Ga), umfassend Methanol, Propylenoxid und Wasser, abgetrennt wird und
(b) aus dem Gemisch (Ga) Propylenoxid unter Erhalt des Gemisches (Gii) abgetrennt wird.

8. Verfahren nach Anspruch 7, **dadurch gekennzeichnet, daß** die Abtrennungen gemäß (a) und (b) destillativ bei
(a) einem Druck im Bereich von 0,2 bis 25 bar in einer Kolonne mit mindestens 5 theoretischen Böden und
(b) einem Druck im Bereich von 0,3 bis 10 bar in einer Kolonne mit mindestens 20 theoretischen Böden
erfolgen.

9. Verfahren nach einem der Ansprüche 1 bis 8, **dadurch gekennzeichnet, daß** zur Herstellung des Propylenoxides ein Zeolithkatalysator, bevorzugt ein Titansilikalit-Katalysator und insbesondere ein Titansilikalit-Katalysator der Struktur TS-1 eingesetzt wird.

## Claims

1. A process for the preparation of propylene oxide in the presence of methanol, in which
(i) in at least one reaction step, propylene oxide is prepared from propene and hydrogen peroxide in the presence of methanol, giving a mixture (Gi) comprising methanol, propylene oxide and water,
(ii) the propylene oxide is separated from the mixture (Gi), giving a mixture (Gii) comprising methanol and water,
(iii)water is separated from the mixture (Gii), giving a mixture (Giii) comprising methanol and methyl formate,
(iv) methanol is separated from the mixture (Giii), and
(v) the methanol separated off in (iv) is fed back into (i).

2. The process according to claim 1, wherein the separation of the methanol from the mixture (Giii) comprising methanol and methyl formate is carried out in a distillation column having at least 5 theoretical plates at pressures in the range from 0.2 to 50 bar.

3. The process according to claim 1 or 2, wherein the methanol separated off in (iv) in the course of the work-up has a methyl formate content of less than 500 ppm.

4. The process according to one of claims 1 to 3, wherein the separation of water in (iii) from the mixture (Gii) is carried out by distillation using a distillation column which has at least 5 theoretical plates, the distillation being carried out at pressures in the range from 0.5 to 40 bar.

5. The process according to one of claims 1 to 3, wherein the water is removed by distillation in (iii), where
(w) a mixture (Gw) comprising principally methanol and methyl formate is separated from the mixture (Gii) at the top of a first distillation column,
(x) the mixture obtained at the bottom of the first distillation column is used as feed for a second distillation column,
(y) a mixture (Gy) comprising principally methanol and methyl formate is obtained at the top of the second distillation column, and
(z) the mixtures (Gw) and (Gy) are combined to give the mixture (Giii).

6. The process according to one of claims 1 to 5, wherein the propylene oxide prepared in (i) is separated by distillation from the mixture (Gi).

7. The process according to one of claims 1 to 5, wherein the separation of the propylene oxide in (ii) is carried out in at least two steps (a) and (b), where
(a) propene is separated from the mixture (Gi), which, in addition to methanol, propylene oxide and water, comprises propene which has not reacted in (i), giving a mixture (Ga) comprising methanol, propylene oxide and water, and
(b) propylene oxide is separated from the mixture (Ga) to give the mixture (Gii).

8. The process according to claim 7, wherein the separations in (a) and (b) are carried out by distillation at
(a) a pressure in the range from 0.2 to 25 bar in a column having at least 5 theoretical plates, and
(b) a pressure in the range from 0.3 to 10 bar in a column having at least 20 theoretical plates.

9. The process according to one of claims 1 to 8, wherein the propylene oxide is prepared using a zeolite catalyst, preferably a titanium silicalite catalyst and in particular a titanium silicalite catalyst having the TS-1 structure.

## Revendications

1. Procédé de fabrication d'oxyde de propylène en présence de méthanol, selon lequel
(i) lors d'au moins une étape de réaction, l'oxyde de propylène est fabriqué à partir de propène et de peroxyde d'hydrogène en présence de méthanol, un mélange (Gi) qui comprend du méthanol, de l'oxyde de propylène et de l'eau étant obtenu,
(ii) l'oxyde de propylène est séparé du mélange (Gi), un mélange (Gii) qui comprend du méthanol et de l'eau étant obtenu,
(iii) l'eau est séparée du mélange (Gii), un mélange (Giii) qui comprend du méthanol et du formiate de méthyle étant obtenu,
(iv) le méthanol est séparé du mélange (Giii) et
(v) le méthanol séparé selon (iv) est recyclé dans (i).

2. Procédé selon la revendication 1, **caractérisé en ce que** la séparation du méthanol du mélange (Giii), qui comprend du méthanol et du formiate de méthyle, est réalisée dans une colonne de distillation qui comprend au moins 5 plateaux théoriques à des pressions dans la plage allant de 0,2 à 50 bars.

3. Procédé selon la revendication 1 ou 2, **caractérisé en ce que** le méthanol séparé selon (iv) dans le cadre du traitement présente une teneur en formiate de méthyle inférieure à 500 ppm.

4. Procédé selon l'une quelconque des revendications 1 à 3, **caractérisé en ce que** la séparation de l'eau du mélange (Gii) selon (iii) a lieu par distillation, une colonne de distillation qui comprend au moins 5 plateaux théoriques étant utilisée et la distillation étant réalisée à des pressions dans la plage allant de 0,5 à 40 bars.

5. Procédé selon l'une quelconque des revendications 1 à 3, **caractérisé en ce que** lors de la séparation par distillation de l'eau selon (iii),
(w) à partir du mélange (Gii), un mélange (Gw) qui comprend essentiellement du méthanol et du formiate de méthyle est séparé à la tête d'une première colonne de distillation,
(x) le mélange obtenu au fond de la première colonne de distillation est introduit en tant qu'alimentation d'une seconde colonne de distillation,
(y) un mélange (Gy) qui comprend essentiellement du méthanol et du formiate de méthyle est obtenu à la tête de la seconde colonne de distillation et
(z) les mélanges (Gw) et (Gy) sont réunis pour obtenir le mélange (Giii).

6. Procédé selon l'une quelconque des revendications 1 à 5, **caractérisé en ce que** l'oxyde de propylène fabriqué en (i) est séparé par distillation à partir du mélange (Gi).

7. Procédé selon l'une quelconque des revendications 1 à 5, **caractérisé en ce que** la séparation de l'oxyde de propylène selon (ii) a lieu en au moins deux étapes (a) et (b), selon lesquelles
(a) à partir du mélange (Gi), qui contient, en plus du méthanol, de l'oxyde de propylène et de l'eau, le propène non réagi en (i), le propène est séparé pour obtenir un mélange (Ga) qui comprend du méthanol, de l'oxyde de propylène et de l'eau et
(b) à partir du mélange (Ga), l'oxyde de propylène est séparé pour obtenir le mélange (Gii).

8. Procédé selon la revendication 7, **caractérisé en ce que** les séparations selon (a) et (b) ont lieu par distillation à
(a) une pression dans la plage allant de 0,2 à 25 bars dans une colonne qui comprend au moins 5 plateaux théoriques et
(b) une pression dans la plage allant de 0,3 à 10 bars dans une colonne qui comprend au moins 20 plateaux théoriques.

9. Procédé selon l'une quelconque des revendications 1 à 8, **caractérisé en ce qu'**un catalyseur à base de zéolithe, de préférence un catalyseur à base de silicalite de titane et notamment un catalyseur à base de silicalite de titane de structure TS-1, est utilisé pour la fabrication de l'oxyde de propylène.
